# EUROPÄISCHE PATENTSCHRIFT

(11) **EP 0 722 935 B1**
(45) Veröffentlichungstag und Bekanntmachung des Hinweises auf die Patenterteilung: **03.03.1999**
(21) Anmeldenummer: 95120134.2
(22) Anmeldetag: 20.12.1995
(51) Int. Cl.: C07D 223/22

(54) **Verfahren zur Reinigung von 5H-Dibenzo-(b,f)azepin**
Process for the purification of 5H-dibenzo-(b.f)-azepine
Procédé de purification de 5H-dibenzo-(b,f)-azépine

(30) Priorität: 27.12.1994 AT 2408/94
(43) Veröffentlichungstag der Anmeldung: 24.07.1996
(73) Patentinhaber: DSM Chemie Linz GmbH, 4021 Linz (AT)
(72) Erfinder: Eichberger, Günter, Dr., A-4512 Weisskirchen (AT); Raml, Walter, Dr., A-4202 Hellmonsödt (AT)
(74) Vertreter: Kunz, Ekkehard, Dr.

(56) Entgegenhaltungen:
- EP-A- 0 237 952
- EP-A- 0 396 134
- CH-A- 442 319
- FR-A- 1 522 192

## Beschreibung

5H-Dibenzo-(b,f)-azepin, allgemein bekannt unter der Bezeichnung Iminostilben, ist ein Zwischenprodukt für pharmazeutisch wirksame Substanzen, wie etwa Carbamazepin.

Die Herstellung von Iminostilben erfolgt durch katalytische Dehydrierung von 10,11-Dihydro-5H-dibenzo-(b,f)-azepin, bekannt unter der Bezeichnung Iminodibenzyl. Insbesondere bei der katalytischen Dehydrierung in der flüssigen Phase oder in der Schmelze, wie beispielsweise in EP 0 237 952 oder EP 0 396 134 beschrieben, weist Iminostilben jedoch eine Vielzahl von Verunreinigungen, wie beispielsweise ein Dimerisierungsprodukt von Iminostilben oder Acridine auf, die bei einer Weiterverarbeitung zu Carbamazepin nicht mehr zu entfernen sind.

Aufgabe der vorliegenden Erfindung war es demnach ein geeignetes Verfahren zur Reinigung von Iminostilben zu finden, mit dem eine ausreichend hohe Reinheit, bei gleichzeitigem Vermeiden von Ausbeuteverlusten, gewährleistet wird. Unerwarteterweise konnte diese Aufgabe durch die Kombination zweier Reinigungsschritte gelöst werden.

Gegenstand der Erfindung ist ein Verfahren zur Reinigung von 5H-Dibenzo-(b,f)-azepin, das dadurch gekennzeichnet ist, daß 5H-Dibenzo-(b,f)-azepin aus einem durch katalytische Dehydrierung von 10, 11-Dihydro-5H-Dibenzo-(b,f)-azepin erhaltenen Reaktionsgemisch im 1. Schritt unter reduziertem Druck zwischen 10 und 100 mbar verdampft und fest abgeschieden wird, das abgeschiedene Produkt im 2. Schritt in einem aromatischen Kohlenwasserstoff aufgeschlämmt und anschließend reines 5H-Dibenzo-(b,f)-azepin isoliert wird.

Wird Iminostilben beispielsweise analog EP 0 237 952 oder EP 0 396 134 hergestellt, so enthält eine durch katalytische Dehydrierung von 10, 11-Dihydro5H-Dibenzo-(b,f)-azepin erhaltene Reaktionsmischung, mit beispielsweise Acridinen und Dimerisationsprodukten verunreinigtes 5H-Dibenzo-(b,f)-azepin (Iminostilben), unumgesetztes 10,11-Dihydro-5H-Dibenzo-(b,f)-azepin, reagierten und überschüssigen Wasserstoffakzeptor, einen Katalysator und gegebenenfalls ein Lösungsmittel, zum Beispiel, wie in EP 0 396 134 angeführt, ein oder mehrere hochsiedende polare protische Lösungsmittel, gegebenenfalls in Mischung mit einem oder mehreren apolaren Lösungsmitteln.

Bevor Iminostilben nach dem erfindungsgemäßen Verfahren gereinigt wird, ist es für die Wiederverwertbarkeit des Katalysators von Vorteil, den Katalysator, wie in EP 0 237 952 und EP 0 396 134 beschrieben, gegebenenfalls durch vorheriges Auflösen der Schmelze in einem geeigneten Lösungsmittel, wie etwa Aceton, Chloroform, Dimethylsulfoxid, Dimethylformamid und/ oder einem oder mehreren Alkoholen, abzufiltrieren.

Im 1. Schritt des erfindungsgemäßen Verfahrens zur Reinigung von Iminostilben, wird gegebenenfalls zuerst das Lösungsmittel aus dem oben beschriebenen Reaktionsgemisch, gegebenenfalls unter reduziertem Druck, beispielsweise über eine Vigreuxkolonne abdestilliert. Anschließend wird Iminostilben aus dem noch verbleibenden Reaktionsgemisch unter reduziertem Druck verdampft und fest abgeschieden, beispielsweise über einen Kratzkühler.
Der Druck kann dabei, je nach Zusammensetzung des Reaktionsgemisches, variieren und liegt bevorzugt zwischen 10 und 100 mbar.
Die Temperatur kann ebenfalls, je nach Zusammensetzung des Reaktionsgemisches variieren und liegt bevorzugt zwischen 120 und 260°C.

Das so gewonnene Produkt wird dann im 2. Schritt gegebenenfalls etwa durch Verreiben homogenisiert, in einem aromatischen Kohlenwasserstoff, wie etwa Toluol oder Xylol, bevorzugt in Toluol, aufgeschlämmt und einige Minuten, bevorzugt 10 bis 20 Minuten, gerührt.
Anschließend wird das so gereinigte Iminostilben durch Absaugen oder Abfiltrieren isoliert, mit dem gleichen Aufschlämmittel, wie im zweiten Reinigungsschritt verwendet wird, und gewünschtenfalls mit Petrolether gewaschen und bei etwa 40 - 70°C im Vakuum getrocknet.
Die dabei erhaltene Mutterlauge kann recycliert und beispielsweise wieder im zweiten Reinigungsschritt als Aufschlämmittel eingesetzt werden.

Durch das erfindungsgemäße Verfahren wird Iminostilben in hoher Reinheit, mit einem mittels Gaschromatographie ermittelten Gehalt von über 99 Flächen-%, ohne störende Verunreinigungen und ohne große Ausbeuteverluste erhalten. Das so gereinigte Iminostilben eignet sich daher besonders zur Weiterverarbeitung zu pharmazeutischen Wirkstoffen, wie Carbamazepin.

### Beispiel 1 - 3:

In einem 500 ml Vierhalsrundkolben wurden 150 g (0,77 mol) 10,11-Dihydro-5H-Dibenzo-(b,f)-azepin, 100 ml (0,85 mol) o-Nitrotoluol, 9 ml eines Lösungsmittels (Tabelle 1) und 9 g 5 % Pd/C vorgelegt, mit N₂ gespült und innerhalb 20 - 25 Minuten auf 235 °C erhitzt. Nach etwa 2 Stunden, der Reaktionsverlauf wurde mittels GC verfolgt, wurde bei 235 - 240°C mit 100 ml o-Nitrotoluol verdünnt und der Katalysator abgesaugt.
Anschließend wurde das Lösungsmittel und überschüssiges Nitrotoluol bei einem Druck zwischen 90 und 30 mbar abdestilliert. Die Kopftemperatur lag zwischen 150 - 170°C und die Sumpftemperatur lag zwischen 150 - 219°C. Iminostilben wurde dann bei einem Druck zwischen 30 und 15 mbar verdampft und als Feststoff abgeschieden. Die Kopftemperatur lag dabei zwischen 180 - 219°C, die Sumpftemperatur zwischen 216 - 240°C.

Der so erhaltene Feststoff wurde in einer Reibschale zerkleinert, in 100 ml Toluol aufgeschlämmt und 15 Minuten gerührt. Dann wurde das Produkt abgesaugt, mit 20 ml Toluol, sowie 50 ml Petrolether nachgewaschen und bei 60°C im Vakuum getrocknet.

In Tabelle 1 sind die Ausbeuten, die ohne Recyclieren der Mutterlauge erhalten wurden, sowie die Lösungsmittel der verschiedenen Versuche und der Gehalt an Verunreinigungen angegeben. Die Werte wurden dabei mittels GC (Gaschromatographie) ermittelt.

**Tabelle 1**

| Bsp. | Lösungsmittel | Ausbeute | FL% | FL % IDB | FL % Acridin % | o-Toluidin |
|---|---|---|---|---|---|---|
| 1 | Diethylenglycol | 117 g (78,8 %) | 99,5 | 0,1 | 0,1 | 0,1 |
| 2 | Diethylenglycolmonoethylether | 116,6 g (78,5 %) | 98,8 | 0,9 | <0,1 | <0,1 |
| 3 | Polyethylenglycol 400 | 119,4 g (80,4 %) | 99,6 | 0,2 | <0,1 | <0,1 |
| FL % Flächenprozent ermittelt mit GC IDB Iminodibenzyl | | | | | | |

## Patentansprüche

1. Verfahren zur Reinigung von 5H-Dibenzo-(b,f)-azepin, dadurch gekennzeichnet, daß 5H-Dibenzo-(b,f)-azepin aus einem durch katalytische Dehydrierung von 10, 11-Dihydro-5H-Dibenzo-(b,f)-azepin erhaltenen Reaktionsgemisch im 1. Schritt unter reduziertem Druck zwischen 10 und 100 mbar verdampft und fest abgeschieden wird, das abgeschiedene Produkt im 2. Schritt in einem aromatischen Kohlenwasserstoffe aufgeschlämmt und anschließend reines 5H-Dibenzo-(b,f)-azepin isoliert wird.

2. Verfahren nach Anspruch 1, dadurch gekennzeichnet, daß der 1. Schritt bei einer Temperaturen von 120 - 260°C durchgeführt wird.

3. Verfahren nach Anspruch 1, dadurch gekennzeichnet, daß das abgeschiedene Produkt in Toluol aufgeschlämmt wird.

## Claims

1. Process for the purification of 5H-dibenzo-[b,f]azepine, characterized in that 5H-dibenzo[b,f]azepine is evaporated under reduced pressure between 10 and 100 mbar from a reaction mixture obtained by catalytic dehydrogenation of 10,11-dihydro-5H-dibenzo[b,f]azepine in the 1st step and is deposited as a solid, the deposited product is suspended in an aromatic hydrocarbon in the 2nd step and then pure 5H-dibenzo[b,f]azepine is isolated.

2. Process according to Claim 1, characterized in that the 1st step is carried out at a temperatures of 120-260°C.

3. Process according to Claim 1, characterized in that the deposited product is suspended in toluene.

## Revendications

1. Procédé de purification de 5H-dibenzo-(b,f)-azépine, caractérisé en ce que dans la 1e phase, de la 5H-dibenzo-(b,f)-azépine est vaporisée à pression réduite comprise entre 10 et 100 mbar et séparée sous forme solide, à partir d'un mélange réactionnel obtenu par déshydratation catalytique de 10,11-dihydro-5H-dibenzo-(b,f)-azépine, en ce que le produit séparé est mis en suspension dans un hydrocarbure aromatique au cours de la 2e phase, et en ce qu'ensuite, de la 5H-dibenzo-(b,f)-azépine pure est isolée.

2. Procédé selon la revendication 1, caractérisé en ce que la 1e phase est exécutée à une température de 120-260 °C.

3. Procédé selon la revendication 1, caractérisé en ce que le produit séparé est mis en suspension dans du toluène.
